# EUROPEAN PATENT SPECIFICATION

(11) **EP 3 325 052 B1**
(45) Date of publication and mention of the grant of the patent: **19.02.2020**
(21) Application number: 16745949.4
(22) Date of filing: 20.07.2016
(51) Int. Cl.: A61C 5/62, A61M 5/315, B05C 17/005, B05C 17/01

(54) **A DEVICE COMPRISING A PLUNGER ASSEMBLY, A METHOD OF ASSEMBLING THE DEVICE AND A METHOD OF MAKING THE PLUNGER ASSEMBLY**
VORRICHTUNG MIT EINER KOLBENANORDNUNG, VERFAHREN ZUR MONTAGE DER VORRICHTUNG UND VERFAHREN ZUR HERSTELLUNG DER KOLBENANORDNUNG
DISPOSITIF UN ENSEMBLE PISTON, PROCÉDÉ D'ASSEMBLAGE DE CE DISPOSITIF ET PROCÉDÉ DE FABRICATION DE L'ENSEMBLE PISTON

(30) Priority: 23.07.2015 EP 15177972
(43) Date of publication of application: 30.05.2018
(73) Proprietor: 3M Innovative Properties Company, St. Paul, MN 55133-3427 (US)
(72) Inventor: PAUSER, Helmut, D-82229 Seefeld (DE); PEUKER, Marc, D-82229 Seefeld (DE)
(74) Representative: Bergen, Katja
(86) International application number: PCT/US2016/043079
(87) International publication number: WO 2017/015332

(56) References cited:
- US-A1- 2012 101 452
- US-A1- 2012 160 047
- US-A1- 2013 115 568

## Description

### Field of the Invention

The invention relates to a device for dispensing a material being provided with a plunger assembly, device for dispensing a dental material comprising the plunger assembly, a method of assembling the device and a method of making a plunger assembly.

### Background Art

Dental composite filling materials often contain a predominant amount of fillers, for example quartz, and a relatively small amount of a flowable monomer, and are typically hardenable by exposure to light having wavelengths of between 450 nanometers and 495 nanometers. Dental composite filling materials may be applied in a cavity in a patient's tooth and light-hardened after.

Typically such dental composite filling materials exhibit a relatively high viscosity and therefore typically require high forces for dispensing from a dispensing device. A dispensing device for dispensing dental composite filling materials is for example disclosed in US 7,128,246 B2. Such a device stores a portion of a dental composite filling material which can be extruded by displacing a piston within the device. The device can be coupled to a hand-held dispensing gun providing a relatively high force for displacing the piston via a hand operable leverage. An alternative dispensing device for dental composite filling materials is disclosed in U.S. Design Patent Des. 419,236.

WO 2006/108085 A2 discloses a syringe delivery system for dispensing a highly viscous material through a syringe delivery opening. The system includes a syringe barrel having a delivery opening, a plunger including a threaded shaft that threadably engages the syringe barrel for selectively dispensing a viscous material through the delivery opening, and a plunger gripping member in gripping communication with the plunger that includes means for sealing the threaded shaft of the plunger so as to prevent contamination by foreign matter. The syringe has a sheath that covers the threaded shaft of the plunger so as to hide the plunger beneath the sheath. The sheath provides a sealed environment for the threaded shaft so as to prevent entrance or contamination by foreign matter. US2013/0115568 also discloses a device for dispensing a dental material.

Although a variety of devices for dental composite filling materials are available there is still a need for a device which is relatively inexpensive, which is easy to use and which accounts for hygiene requirements in dentistry.

### Summary of the Invention

The invention is directed to a device for dispensing a material according to claim 1. The material may be a dental material or another flowable, preferably pasty material. The device comprises a plunger assembly. The plunger assembly has a screw which comprises a first thread portion and a second thread portion. The first and second thread portion differ in shape. Further the first and second thread portion, in combination, form one continuous screw thread, the plunger assembly further comprising a nut which engages the screw thread by a nut thread, and wherein the nut thread corresponds in shape to a negative shape of the first thread portion.

The invention is advantageous in that it provides for a facilitated assembly of the plunger and a facilitated assembly of the plunger assembly with a device for dispensing a dental material. The invention further is advantageous in that it provides a device that allows for dispensation of dental material (particularly a dental composite filling material) in the form of a pre-dosed portion. Thus the use of a separate instrument for separating (for example cutting or scraping off) a portion of dental composite filling material from the device is not required. Further the device preferably allows for dispensation of the dental material at a precisely controlled manner and particularly may exhibit minimized afterflow (or run on) of dental composite filling material after suspending dispensation. Although a separate instrument may be used for picking up the pre-dosed portion, any intense interaction (like cutting, abrading, scraping) between the instrument and the device can be minimized. This further helps avoiding bringing any particles abraded or cut from the device into the dental composite filling material due to an interaction between the device and the dental instrument. Further the device is advantageous because it helps providing for a convenient handling, precise dosage and hygienic storage of the dental material.

The continuous screw thread preferably is an outer thread and the nut thread is preferably an inner thread. Preferably the continuous screw thread is configured so that the nut can be continuously screwed from the first and second thread portion (including the transition between the first and second thread portion) and vice versa.

The nut thread corresponds to an impression (or negative replication) of the first thread portion of the screw thread or at least a portion of the first thread portion. In other words, the nut thread corresponds in shape to the negative shape of the first thread portion or at least a portion of the first thread portion. Further, the nut thread differs in shape from the negative shape of the second thread portion.

In a further embodiment the nut and the screw are displaceable relative to each other between a first position in which the nut engages only the first thread portion and a second position in which the nut engages only the second thread portion. The first thread portion may form an end portion of the screw thread. The nut may be configured such that it entirely covers the first portion in the first position. Further, the second thread portion preferably extends over a greater length than the first portion. The length is measured parallel to a longitudinal axis of the screw. The longitudinal axis further is preferably an axis of symmetry or center axis of the screw thread. The longitudinal axis may also correspond to the rotation axis about which the screw and the nut are rotatable relative to each other in use of the invention.

The difference in shape is based on a retention structure in the first thread portion which is absent in the second thread portion. In particular, the first thread portion may have an additional positive structure (like a protrusion) which is replicated as a negative structure (like a recess) in the nut, and the additional structure is preferable absent in the second thread portion.

In a particular embodiment retention structure is a web which protrudes into the thread groove of the first thread portion. However, the skilled person will recognize that other structures can be used, like for example bulged out portions on the thread, in the groove or combinations thereof.

The retention structure is provided by a taper thread forming at least part of the first thread portion. The taper thread has a greater diameter and smaller diameter and tapers between the greater and the smaller diameter. The taper may be linear or curved. The smaller diameter of the taper thread preferably corresponds to the diameter of the thread of the second thread portion. The first thread portion is preferably arranged with its smaller diameter directly adjacent the second thread portion. The retention in this case is provided by a press fit between the nut and the taper thread. Once the nut and the screw are positioned further toward the second position the press fit and, accordingly, also the retention is suspended.

A thread diameter as referred to herein refers to the nominal thread diameter, meaning the outer diameter of an outer thread. It is noted that the inner diameter of an inner thread in contrast is a core diameter which does not correspond to the nominal diameter.

The first and second thread portion are preferably based on the same pitch and thread profile. The second thread portion is preferably formed by an outer straight thread. Further, the thread of the first thread portion and the thread of the second thread portion continue into one another. The first thread portion and the second thread portion are preferably arranged directly adjacent to each other.

In one embodiment the screw has a seal adjacent the first thread portion. Further the screw preferably has a handle adjacent the second thread portion. Thus, the seal and the handle portion are preferably arranged on opposite sides of the first and second thread portion. In other words the first and second thread portion are arranged between the seal and the handle portion. The seal and the handle portion may each form an end of the screw. Further, each of the seal and the handle are greater in outer diameter than the outer diameter of the screw thread. A seal having a greater diameter than the outer thread of the screw helps preventing the thread to be contaminated with a dental material when the screw is screwed into a container for expelling the dental material. Such contamination may cause additional friction so that preventing the contamination helps minimizing the torque required to rotate the screw into the container.

In one embodiment the seal is formed as a conical lip seal. Such a conical lip seal is preferably arranged with its wider end toward the dental material. Thus, urging the dental material forward typically causes the seal to be urged for further widening so that the seal with a container wall typically increases with the urging the dental material at increased forces.

Preferably each of the screw and the nut are monolithically formed. Preferably, the screw and the nut are molded from a plastic material. Each of the screw and the nut may be formed in one piece by injection molding from a plastic material. This means that the nut as well as the screw are preferably each formed in its final shape from a molten plastic material and not pre-manufactured in two or multiple components which are assembled after.

A suitable plastic material for the screw may be selected from among polybutadiene terephthalate (PBT), polyamide (PA), polyoxymethylene (POM). A preferred plastic material for the screw is PBT. A suitable plastic material for the nut may be selected from among polypropylene (PP), polyethylene (PE). The plastic materials listed may be glass-fiber reinforced. A preferred plastic material for the nut is glass-fiber reinforced PP.

In a preferred embodiment the nut forms an overmold on the screw. A teaching for obtaining such an overmold is provided further below with respect to the method of making a plunger assembly.

In one embodiment the nut is made from glass-fiber reinforced material. The glass-fiber reinforced material preferably has a higher glass-fiber content per weight than the screw. However, the screw preferably has a glass-fiber content per weight of zero. It has been found that by the glass-fiber content not only the material strength can be controlled. Rather, the shrinkage characteristics of the nut after being molded from the glass-fiber material can be controlled by the glass-fiber content. Generally, the glass-fiber reinforced material has a lower shrinkage rate as it hardens from a liquid/molten material. This allows for example to control the fit of the nut on the screw between a lose fit and a press fit. A lose fit may for example be achieved by molding the nut on the screw shortly after molding the screw. The term "shortly after" refers to a time period after which the screw has hardened but before all of the shrinkage has completed. Typically, such a time period is up to 12 hours after molding. For achieving a lose fit the nut may be molded from a material which has a relatively low shrinkage rate (for example a glass-fiber reinforced material) while the screw may be molded from a material having a comparably high shrinkage rate. It is however preferred that a press fit is established between the nut and the screw, and the quality of the press fit can be controlled by the material combination of the nut and the screw, in particular by the glass-fiber content of the material used for molding the nut. Any glass-fiber reinforcement can be applied for the plastic materials mentioned above.

In a further embodiment the device preferably further comprises a container having a front end with a dispensing opening and an opposite rear end with a rear opening. The plunger assembly is preferably attached or attachable with the nut in the container.

In an embodiment the nut of the plunger assembly has an outer thread. The outer thread of the nut is configured for engaging with an inner thread of the container. If the plunger assembly is attached to the container the outer thread of the nut engages with the inner thread of the container. The container preferably forms an inner chamber extending between the dispensing opening and the rear opening. The inner chamber extends along a chamber length, and the inner container thread extends only over a part of the container length. The inner chamber accordingly preferably has a first chamber portion and a second chamber portion. The first chamber portion is preferably configured to store the dental material. Further, the second chamber portion preferably forms the inner container thread. In more particular, the container may have a side wall that forms the first chamber portion having a cylindrical inner surface and the second chamber portion having the inner container thread. The container wall preferably has a stepped configuration. Preferably, the step is formed between the first and second chamber portion. Preferably, the core diameter of the inner container thread is equal or greater than the inner diameter of the first chamber portion. Therefore the step is formed by the difference in diameter of the first chamber portion and the major thread diameter (= diameter which essentially corresponds to the outer diameter of the screw thread) of the inner container thread of the second chamber portion. Thus, the screw of the plunger assembly of the device of the invention does not interfere or collide with the inner container thread during assembly or use of the device. Further, the nut is prevented from penetrating into the first chamber portion by the step provided by the difference in diameter.

The inner chamber is preferably closed at the front end of the container with a front wall of the container, through which a passageway extends between the inner chamber and the dispensing opening.

The device may further comprise a cover within which the container may be receivable or received. The cover and the container are preferably rotatable relative to each other about the longitudinal axis. The cover and the container may be configured and arranged relative to each other to form a rotary slide valve in combination. Therefore, the cover has an opening which can be selectively aligned with or positioned away from the dispensing opening of the container. Accordingly, the rotary slide valve is operable by rotation (of the cover and the container relative to each other) about the longitudinal axis between a storage position and a dispensing position. In the storage position the cover closes the dispensing opening of the container, whereas in the dispensing position the cover opening opens the dispensing opening of the container. In the storage position the cover opening and the dispensing opining of the container thus are preferably offset and do not overlap with each other. At least the cover at the opening may comprise a sharp cutting edge delimiting at least a portion of the cover opening. The cutting edge is preferably arranged such that during rotation of the rotary slide valve from the dispensing position toward the storage position the cutting edge penetrates through dental material extruded from the container and protruding from the dispensing opining. The cutting edge may have a wedge shaped profile extending over a part of or entirely over the circumference of the cover opening with the wedge tapering off toward the cover opening.

Any rotation of the container and the cover relative to each other is preferably performed by turning the screw and the cover relative to each other. In a preferred embodiment the device is adapted such that the relative rotation between the cover and the container is enabled within a predetermined rotation angle and restricted from a rotation outside that predetermined rotation angle. The rotation angle between the storage position and the dispensing position corresponds to the predetermined rotation angle. Further, the device is adapted such that a rotation direction of the screw and the container relative to each other for extruding dental material from the container corresponds to a rotation direction of the container and the cover which urges the rotary slide valve in a direction from the storage toward the dispensing position. Thus, an initially closed device (valve in storage position) automatically opens upon operating the screw for dispensing the dental material.

In a further embodiment the device comprises the dental material, in particular a dental composite filling material, and in more particular a light hardenable dental composite filling material. Dental composite filling materials are typically characterized by at least one or all of the following features:
a) radiation curable (especially within the region of visible light, in particular blue light);
b) formulated as a one-component composition (in contrast to e.g. dental impression materials which are formulated as two-component compositions which are mixed from a base paste and a catalyst paste shortly prior to use);
c) highly viscous;
d) slightly tacky, for example sticky if touched with the fingers;
e) "cuttable", for example can be cut into pieces with a dental instrument or knife (scalpel);
f) "firm" for example shapeable by applying manual forces but generally self-supporting in absence of such forces at least over a time period of about 5 minutes. A combination of the features (a), (b), (c) and (d) is sometimes preferred.

Dental composite materials typically comprise a hardenable resin matrix comprising hardenable components, an initiator system suitable to harden the hardenable components contained in the resin matrix and filler(s). The filler content is typically above about 50, 60 or 70 wt.-% with respect to the weight of the dental composite material. Typical ranges include from about 50 to about 90 wt.-% or from about 60 to about 80 wt.-%. The hardenable components typically comprise unsaturated moieties (carbon-carbon unsaturation) like (meth)acrylate moieties. In order to be crosslinkable, the hardenable components typically comprise at least about 2 unsaturated moieties.

The composite material may comprise only one type of filler or different types of fillers. Suitable filler(s) include fumed silica, quartz, ground glasses, non-water-soluble fluorides such as CaF₂, silica gels such as silicic acid, in particular pyrogenic silicic acid and granulates thereof, cristobalite, calcium silicate, zirconium silicate, zeolites, including the molecular sieves, barium sulphate and/or yttrium fluoride. Suitable fumed silicas include for example, products sold under the tradename AerosilTM series OX-50, -130, -150, and - 200, Aerosil R8200 available from Degussa AG, (Hanau, Germany), CAB-O-SILTM M5 available from Cabot Corp (Tuscola, 111.), and HDK types, e.g. HDK-H 2000, HDK H15; HDK H18, HDK H20 and HDK H30 available from Wacker. The average surface area of the silica particles is preferably greater than about 15 m²/g more preferably greater than about 30 m²/g.

A "hardenable component or material" or "polymerizable component" is any component which can be cured or solidified e.g. by radiation-induced polymerization. A hardenable component may contain only one, two, three or more polymerizable groups. Typical examples of polymerizable groups include unsaturated carbon groups, such as a vinyl group being present i.a. in a (methyl)acrylate group.

"(Meth)acryl" is a shorthand term referring to "acryl" and/or "methacryl". For example, a "(meth) acryloxy" group is a shorthand term referring to either an acryloxy group (i. e.,CH₂=CH-C(O)-O-) and/or a methacryloxy group (i. e., CH₂=C(CH₃)-C(O)-O-).

A "curing, hardening or setting reaction" is used interchangeable and refers to a reaction wherein physical properties such as viscosity and hardness of a composition changes over the time due to a chemical reaction between the individual components.

"Light hardenable" shall mean that the composition can be cured by applying radiation with light, preferably radiation with light at a wavelength within the visible light spectrum under ambient conditions (for example approximately 23 ± 10 degrees Celsius) and within a reasonable time frame (e.g. within about 15, 10 or 5 minutes).

The term "visible light" is used to refer to light having a wavelength of about 380 to about 750 nanometers (nm).

Further the device may comprise a tooth color encoding indicating the tooth color which the color of the dental material contained in the device approximately corresponds to. The tooth color encoding may relate to a particular material class encoding. The device may comprise both, the tooth color encoding and the material class encoding. In one example the tooth color encoding corresponds to a encoding of the so-called VITA shade guide. The VITA shade guide provides certain predetermined tooth color shades indicated by a encoding, for example the encodings B1, A1, B2, D2, A2, C1, C2, D4, A3, D3, B3, A3.5, B4, C3, A4, C4, each being representative of a certain tooth color.

The invention further relates to a method of assembling a device for dispensing material. The method comprises the steps of:
- providing the device of the invention;
- providing the container (as described herein);
- turning the plunger assembly and/or the container relative to each other and thereby causing the nut and the container to screw into each other;
- upon the nut and the container being blocked from further rotation as the nut and the step contact each other, further turning the plunger assembly and/or the container relative other and thereby causing the screw and the nut to screw relative to each other.

The device of the invention is preferably provided with the plunger assembly in the first position in which the nut is only positioned on the first thread portion. In this position the nut and the screw are preferably retained against relative rotation to each other. The retention is provided by means of the retention structure. In one embodiment the retention is provided by the nut in combination with the taper thread in the first position of the nut and the screw.

In the step of turning the plunger assembly and/or the container relative to each other the plunger assembly and the container are preferably also moved toward each other. Such movement preferably has a feed which corresponds to the relative rotation speed between the plunger assembly and the container multiplied by the pitch of the outer thread of the screw. For the assembly of the plunger assembly and the container an automatic screwing device may be used.

In a further aspect the invention relates to a method of making the plunger assembly.

The method comprises the steps of:
- molding a screw comprising a first thread portion and a second thread portion, the first and second thread portion differ in shape but form in combination one continuous screw thread; and
- overmolding the first thread portion and thereby forming a nut embracing (or entirely surrounding) the screw.

The screw and the nut correspond to the screw and the nut of the plunger assembly of the device of the invention and may be provided in the embodiments as disclosed herein.

In the step of overmolding a molten plastic material (as defined herein) is preferably molded entirely circumferentially around the screw. Further, a step of cooling the molded screw is preferably provided between the step of molding the screw and the step of overmolding the first thread portion of the screw.

The skilled person will recognize that it may be possible to mold the nut in a first step and molding the screw into the pre-molded nut to achieve a similar assembly, although such method is not preferred.

### Brief Description of the Figures

- Fig. 1: is a side view of a device according to an embodiment of the invention;
- Fig. 2: is a perspective view of the device of Fig. 1 in use;
- Fig. 3: is a perspective view of a device according to a further embodiment of the invention;
- Fig. 4: is an exploded view of a device according to an embodiment of the invention;
- Fig. 5: is a partial view of a plunger assembly according to an embodiment of the invention;
- Fig. 6: is a partial view of a plunger assembly according to a further embodiment of the invention; and
- Fig. 7: is a partial view of a plunger assembly according to still a further embodiment of the invention.

### Detailed Description of the Invention

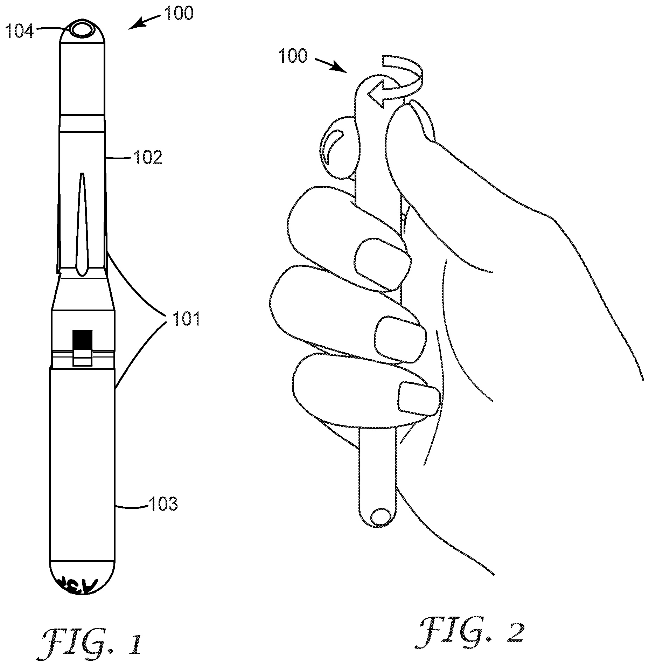

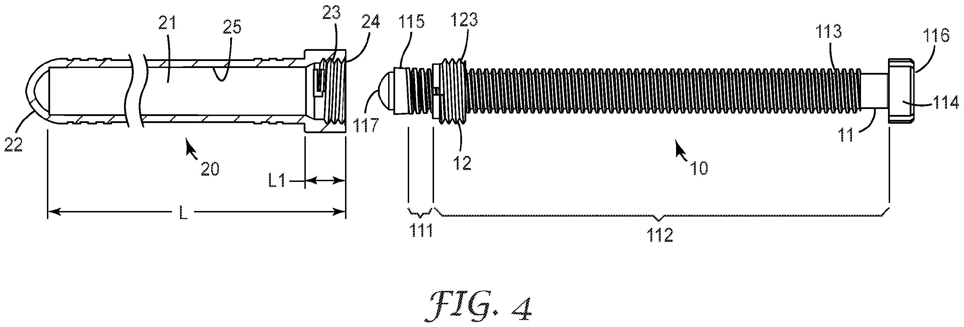

Fig. 1 shows an exemplary device 100 for dispensing a dental material as it may be used with the present invention. A similar device is disclosed in detail in WO 2014/179219 A1. The device 100 has a housing 101 which is formed of a cover 102 and a handle 103. The housing 101 houses a container in which the dental material is stored and within which a thrust screw or screw plunger (also referred to as "screw" herein) is accommodated for extruding the dental material from the container. Such a container and screw are described in further detail in Figures 4 ff. herein. For dispensing of the dental material the cover 102 has an opening 104 which can be selectively brought in alignment with an outlet for the dental material in the container by rotation of the cover 102 relative to the container. For closing the device 100 the cover 102 and the container can be rotated to misalign the opening 104 and the container outlet so that a wall of the cover 102 blocks the outlet. Accordingly, the cover 102 and the container of this example form a rotary slide valve in combination. In the example shown the opening 104 is closed. The handle 103 and the cover 102 are rotatably interconnected with each other so that dental material can be extruded from the device 100 by rotating the handle 103 and the cover 102 against each other. The device 100 is further adapted such that upon rotating the handle 103 and the cover 102 against each other the initially closed device 100 opens automatically. Therefore, the device 100 is adapted such that a rotation of the handle 103 and the cover 102 against each other first causes the closed device to automatically open, and a further rotation of the handle 103 and the cover 102 against each other causes dental material to be dispensed.

The device 100 is further adapted such that a rotation of the handle 103 and the cover 102 in the opposite direction causes the device 100 to close. Thereby any material extending through the valve is sheared off. In Fig. 2 the device 100 is shown in operation by a user.

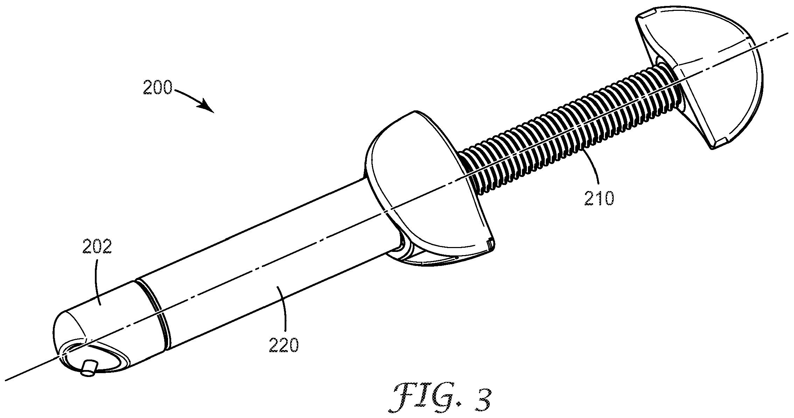

Fig. 3 shows an alternative device 200 as it may be used with the present invention. The device 200 has a screw plunger 210 which is received within a container 220. The container 220 stores a dental material which can be extruded through an opening 204 in the container 220. In the example the device 200 has a rotatable closure cap 202 for selectively opening or closing the device 200. The skilled person will recognize other devices which may be used with the present invention, like for example a screw syringe with a removable cap.

Fig. 4 illustrates the plunger assembly 10 for the device of the of the invention. The plunger assembly 10 has a screw 11 and a nut 12. The screw 11 has an outer thread 113 and the nut 12 has an inner thread (not visible in this view) engaging with the outer thread 113 of the screw 11. The screw 11 in particular has a first thread portion 111 and a second thread portion 112. The first and second thread portion 111, 112 differ in shape, as further explained in detail in the following. In particular, the first thread portion 111 is formed by a taper thread which widens in a direction away from the second thread portion 112. In contrast, the second thread portion is formed by a straight thread. The first and second thread portion 111, 112 seamlessly transition into each other so that the first and second thread portion 111, 112 in combination form common outer thread 113. The pitch of the thread is constant over the first and second portion 111, 112. In the example the pitch is 1 mm. Therefore a full rotation of the screw 11 by 360 degrees relative to the nut 12 causes the screw 11 and the nut 12 to linearly displace by 1 mm. This results in a transmission providing for a relatively high force in the linear direction if rotated at an acceptable torque by hand.

The inner thread of the nut 12 corresponds in shape to a negative shape of the first thread portion. This can be achieved by manufacturing in the following manner:
- providing the screw 11 in a first step; and
- making the nut 12 by overmolding the first thread portion 111 of the screw 11 subsequently.

Thus, the inner thread of the nut 12 is replicated from the outer thread of the screw 11, in particular from the outer thread within the first thread portion. Although such method is preferred for the purpose of the present invention, the skilled person will recognize other methods of making a nut which has an inner thread which corresponds to the negative shape of the screw thread. For example, a nut made of two halves or multiple segments may be provided or a nut which is separately manufactured and screwed onto the screw.

Because the first thread portion 111 differs in shape from the second thread portion 112, the inner thread of the nut 12 replicated from the first thread portion 111 accordingly differs in shape from the negative shape of the second thread portion 112.

In the present example, however, the screw has a handle 114 and a rear end 116 and a seal 115 at a front end 117 of the screw 11. Each of the seal 115 and the handle 114 is enlarged in diameter with respect to the outer thread 113 of the screw. Because the screw 11 is monolithically formed with the seal 115 and the handle 114, screwing the nut 12 from outside on the screw 11 is not enabled.

The nut 12 further has an additional outer thread 123. The outer thread 123 is configured to form a screw connection with an inner thread 23 of the container 20. The container 20 forms an inner chamber 21 extending between a dispensing opening 22 and a rear opening 24 along a length L of the chamber 21. The inner container thread 23 extends only over a partial length L1 of the container length L. Over the remainder of the container length (L - L1) the chamber has an inward chamber surface 25 extending at a generally uniform cross-section, preferably at a circular cross-section. Further, the innermost diameter of the inner container thread 23 is equal or greater than the inner diameter of the chamber surface 25. Thus, the risk of any undesired deformation or damaging of the seal 115 during insertion of the front end 117 of the screw 11 into the container can be minimized.

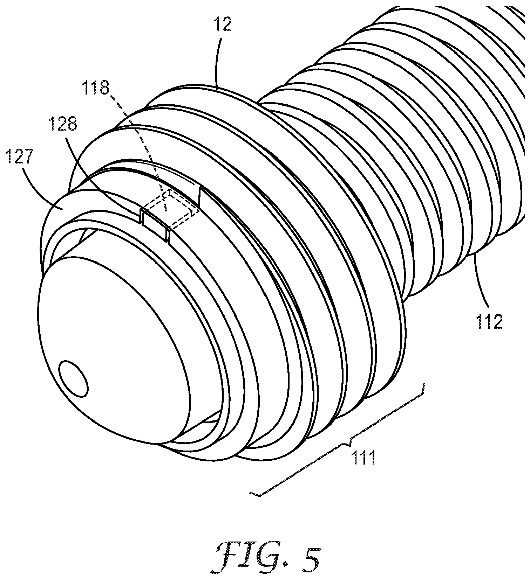

Fig. 5 shows the nut 12 arranged on the first thread portion 111. In this example the first thread portion 111 has a retention structure 118 in the form of a web which protrudes into the thread groove of the first thread portion 111. The inner thread of the nut 12 replicates the retention structure 118 in the form of a corresponding negative retention structure 128, in the example a recess which accommodates the web. Accordingly, the web and the recess engage with each other and hinder the nut 12 and the screw 11 in a rotation relative to each other. A rotation of the nut 12 and the screw 11 relative to each other can however be achieved by urging the nut 12 and the screw 11 relative to each other at a torque which exceeds a certain threshold torque. Upon exceeding the threshold torque the screw 11 and/or the nut 12 typically deforms in the area of and adjacent the retention structure 118 and/or the negative retention structure 128. Thus, the retention provided by the retention structure 118 and negative retention structure 128 can be overcome. The threshold torque can be pre-determined by design of the retention structure 118, for example by the dimensions of the retention structure.

The negative retention structure 128 is arranged at or adjacent a front end 127 of the nut 12. Therefore, a rotation of the nut 12 and the screw 11 relative to each other in a direction which causes the nut 12 to axially move in a direction from the first thread portion 111 toward the second thread portion 112, a relatively small rotation angle provides for the nut 12 to axially move to a position in which the nut 12 is no longer in contact with the retention structure 118. This is advantageous because once the retention between the nut 12 and the screw 11 is overcome so that the nut 12 and the screw 11 rotate relative to each other, the retention structure 118 typically still causes a friction between the nut 12 and the screw 11 (and a corresponding torque required for the rotation) as long as the nut 12 is in contact with the retention structure.

The threshold torque required to break the screw 12 and the screw 11 lose for a rotation relative to each other can be used for assembling the nut 12 in the container 20 (Fig. 4). In particular, the nut 12 is preferably screwed with its outer thread 123 into the inner thread 23 of the container 20 by rotating the screw 11. Due to the retention between the nut 12 and the screw 11 the nut 12 is driven by the screw 11. Therefore, the nut 12 can be screwed into the container without the nut 12 and screw 11 moving relative to each other. However, once the nut 12 reaches the end of the inner thread 23 of the container any further rotation of the nut 12 relative to the container 20 is prevented. At this stage a further rotation of the screw in the same direction causes the threshold torque to be overcome and that threshold torque causes a tightening of the nut 12 within the container at a predetermined torque. Accordingly, for the assembly of the screw 11 and the nut 12 not additional torque control is required. Therefore, the assembly may be performed manually or automatically at the same quality.

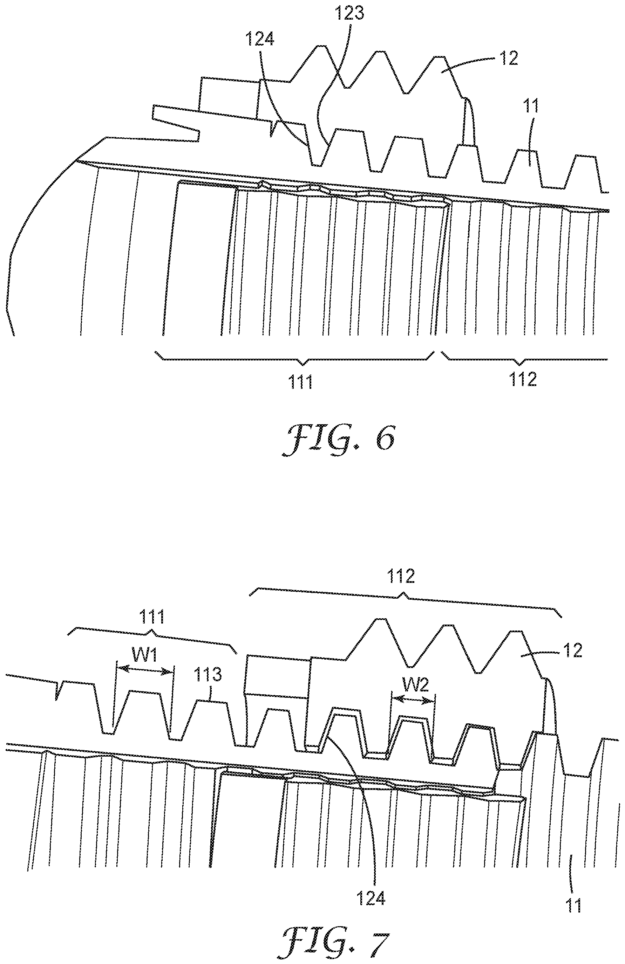

Fig. 6 shows a further embodiment of the screw 11 and the nut 12. For ease of understanding the same reference numbers are used. In the example of Fig. 6 the first thread portion 111 is formed by (or comprises) a taper thread and the second thread portion 112 is formed by a straight thread. The lower diameter of the taper thread of the first thread portion 111 corresponds to the diameter of the straight thread of the second thread portion 112. Further, the lower diameter of the taper thread of the first thread portion 111 is arranged directly adjacent the second thread portion 112, and the taper thread widens in a direction from the second thread portion 112 toward the first thread portion 111.

The inner thread 124 of the nut 12 corresponds in shape to the outer shape of the first thread portion 111. In particular the inner thread 124 is a taper thread which corresponds to a negative of the taper thread of the first thread portion 111. Accordingly, a rotation of the nut 12 and the screw 11 relative to each other to axially move the nut 12 in a direction from the first portion 111 toward the second portion 112 causes the inner thread 124 to establish a lose fit with the outer thread 113 of the first thread portion 111. Therefore, the nut 12 and the screw 11 can be rotated relative to each other at a relatively low torque. A threshold torque required to break the nut 12 and the screw 11 lose from each other may be achieved due to an overmolding of the screw 11 by the nut 12. Typically during overmolding the nut 12 is initially formed by a ring-shaped melt of a plastic material. As the plastic material hardens and cools it typically shrinks onto the screw 11 so that the nut 12 finally forms a press fit on the first thread portion 111. In addition to the press fit a surface adhesion and/or a surface engagement may occur between contacting surfaces of the nut 12 and the screw 11. A surface engagement may be formed for example by a surface roughness present on the screw 11 which replicates as a negative shape of the nut 12, for example. Once the so formed threshold torque is overcome (during a rotation of the nut 12 and the screw 11 relative to each other to axially move the nut 12 in a direction from the first portion 111 toward the second portion 112) the torque to further rotate the nut 12 and the screw 11 rapidly reduces upon a rotation over only small incremental rotation angles because of the two taper threads move away from each other and form a lose fit with each other. Preferably the taper of the taper threads is such that the inner thread 124 at its greatest diameter still engages with the outer thread 113 of the second thread portion 112. Thus, the inner thread 124 of the nut 12 is sufficiently engaged with the outer thread 113 of the second thread portion 112 in a position in which the nut 12 is entirely positioned on only the second thread portion.

The taper thread may be used in combination with the retention structure described in the example of Fig. 5. In this embodiment any press fit, surface adhesion and/or surface engagement by overmolding is optional.

As described above, the threshold torque for breaking the nut 12 and the screw 11 lose from each other can be used for assembly of the nut 12 in the container 20 (Fig. 4).

Fig. 7 shows a further embodiment in which the outer thread 113 of the first portion 111 has a wider thread profile than the outer thread 113 of the second portion 112. Again, a threshold torque may be achieved identically as described in the example of Fig. 6 by overmolding so that the inner thread 124 of the nut 12 forms a tight fit or press fit with the outer thread 113 of the first thread portion 11. In this example, however, the torque required for rotation of the nut 12 and the screw 11 relative to each other only gradually reduces as the nut 12 axially moves toward the second thread portion 112. Only when the nut 12 is positioned entirely on only the second thread portion a full lose fit is formed between the inner thread 124 of the nut 12 and the outer thread 113 of the second thread portion. The example of Figures 5 and/or 6 may be combined with the present example as appropriate.

## Claims

1. A device for dispensing a material, comprising a plunger assembly (10), the plunger assembly having a screw comprising a first thread portion (111) and a second thread portion (112), the first and second thread portion differ in shape and, in combination, form one continuous screw thread, the plunger assembly further comprising a nut (12) which engages the screw thread by a nut thread, and wherein the nut thread corresponds in shape to a negative shape of the first thread portion, wherein the difference in shape is based on a retention structure in the first thread portion which is absent in the second thread portion, and wherein the retention structure is provided by a taper thread forming at least part of the first thread portion.

2. The device of claim 1, wherein the retention structure is a web which protrudes into the thread groove of the first thread portion.

3. The device of any of the preceding claims, wherein the first and second thread portion are based on the same pitch and thread profile, the second thread portion being formed by an outer straight thread, and wherein the thread of the first thread portion and the thread of the second thread portion continue into one another.

4. The device of any of the preceding claims, wherein the screw has a seal adjacent the first thread portion and a handle adjacent the second thread portion, wherein each of the seal and the handle are greater in outer diameter than the outer diameter of the screw thread.

5. The device of any of the preceding claims, wherein each of the screw and the nut are monolithically formed.

6. The device of any of the preceding claims, wherein the screw and the nut are molded from a plastic material and wherein the nut forms an overmold on the screw.

7. The device of claim 6, wherein the nut is made from glass-fiber reinforced material having a higher glass-fiber content per weight than the screw, and wherein the screw preferably has a glass-fiber content per weight of zero.

8. The device of any of the preceding claims, further comprising a container having a front end with a dispensing opening and an opposite rear end with a rear opening, and wherein the plunger assembly being attached with the nut in the container.

9. The device of claim 8, wherein the nut has an outer thread engaging with an inner thread of the container, wherein the container forms an inner chamber extending between the dispensing opening and the rear opening, inner chamber having a first chamber portion and a second chamber portion with the second chamber portion forming the inner container thread, wherein a step is formed between the first and second chamber portion.

10. A method of assembling a device for dispensing material, comprising the steps of:
providing the device of any of the preceding claims;
turning the plunger assembly (10) and/or the container (20) relative to each other and thereby causing the nut (12) and the container (20) to screw into each other;
upon the nut and the container being blocked from further rotation as the nut and the step contact each other, further turning the plunger assembly and/or the container relative other and thereby causing the screw and the nut to screw relative to each other.

11. A method of making a plunger assembly according to any of the preceding claims, comprising the steps of:
molding a screw comprising a first thread portion (111) and a second thread portion(112), the first and second thread portion differ in shape but form in combination one continuous screw thread; and
overmolding the first thread portion and thereby forming a nut (12) embracing the screw.

## Patentansprüche

1. Eine Vorrichtung zur Abgabe eines Materials, umfassend eine Kolbenanordnung (10), wobei die Kolbenanordnung eine Schraube mit einem ersten Gewindeabschnitt (111) und einem zweiten Gewindeabschnitt (112) aufweist, wobei sich der erste und der zweite Gewindeabschnitt in der Form unterscheiden und in Kombination ein durchgehendes Schraubengewinde bilden, wobei die Kolbenanordnung ferner eine Mutter (12) umfasst, die durch ein Muttergewinde mit dem Schraubengewinde in Eingriff steht, und wobei das Muttergewinde in der Form einer negativen Form des ersten Gewindeabschnitts entspricht, wobei der Unterschied in der Form auf einer Haltestruktur in dem ersten Gewindeabschnitt basiert, die in dem zweiten Gewindeabschnitt nicht vorhanden ist, und wobei die Haltestruktur durch ein konisches Gewinde bereitgestellt ist, das zumindest einen Teil des ersten Gewindeabschnitts bildet.

2. Die Vorrichtung nach Anspruch 1, wobei die Haltestruktur ein Steg ist, der in den Gewindegang des ersten Gewindeabschnitts ragt.

3. Die Vorrichtung nach einem der vorstehenden Ansprüche, wobei der erste und der zweite Gewindeabschnitt auf der gleichen Steigung und dem gleichen Gewindeprofil basieren, wobei der zweite Gewindeabschnitt durch ein äußeres gerades Gewinde gebildet wird, und wobei das Gewinde des ersten Gewindeabschnitts und das Gewinde des zweiten Gewindeabschnitts ineinander übergehen.

4. Die Vorrichtung nach einem der vorstehenden Ansprüche, wobei die Schraube eine Dichtung angrenzend an den ersten Gewindeabschnitt und einen Griff angrenzend an den zweiten Gewindeabschnitt aufweist, wobei sowohl die Dichtung als auch der Griff einen größeren Außendurchmesser aufweisen als das Schraubengewinde.

5. Die Vorrichtung nach einem der vorstehenden Ansprüche, wobei sowohl die Schraube als auch die Mutter monolithisch ausgebildet sind.

6. Die Vorrichtung nach einem der vorstehenden Ansprüche, wobei die Schraube und die Mutter aus einem Kunststoffmaterial geformt sind und wobei die Mutter eine Umspritzung an der Schraube bildet.

7. Die Vorrichtung nach Anspruch 6, wobei die Mutter aus glasfaserverstärktem Material mit einem höheren Glasfaseranteil in Bezug auf das Gewicht als die Schraube hergestellt ist, und wobei die Schraube vorzugsweise einen Glasfaseranteil in Bezug auf das Gewicht von Null aufweist.

8. Die Vorrichtung nach einem der vorstehenden Ansprüche, ferner umfassend einen Behälter mit einem vorderen Ende mit einer Ausgabeöffnung und einem entgegengesetzten hinteren Ende mit einer hinteren Öffnung, und wobei die Kolbenanordnung mit der Mutter in dem Behälter befestigt ist.

9. Die Vorrichtung nach Anspruch 8, wobei die Mutter ein Außengewinde aufweist, das mit einem Innengewinde des Behälters in Eingriff steht, wobei der Behälter eine innere Kammer bildet, die sich zwischen der Ausgabeöffnung und der hinteren Öffnung erstreckt, wobei die innere Kammer einen ersten Kammerabschnitt und einen zweiten Kammerabschnitt aufweist, wobei der zweite Kammerabschnitt das Innengewinde des Behälters bildet, wobei eine Stufe zwischen dem ersten und dem zweiten Kammerabschnitt ausgebildet ist.

10. Ein Verfahren zur Montage einer Vorrichtung zur Abgabe eines Materials, umfassend die folgenden Schritte:
Bereitstellen der Vorrichtung nach einem der vorstehenden Ansprüche;
Drehen der Kolbenanordnung (10) und/oder des Behälters (20) relativ zueinander und dadurch Bewirken, dass die Mutter (12) und der Behälter (20) ineinander verschraubt werden;
sobald eine weitere Drehung der Mutter und des Behälters blockiert wird, wenn die Mutter und die Stufe miteinander in Kontakt stehen, weiteres Drehen der Kolbenanordnung und/oder des Behälters relativ andere und dadurch Bewirken, dass die Schraube und die Mutter relativ zueinander verschraubt werden.

11. Ein Verfahren zur Herstellung einer Kolbenanordnung nach einem der vorstehenden Ansprüche, umfassend die folgenden Schritte:
Formen einer Schraube, umfassend einen ersten Gewindeabschnitt (111) und einen zweiten Gewindeabschnitt (112), wobei sich der erste und der zweite Gewindeabschnitt in der Form unterscheiden, aber in Kombination ein durchgehendes Schraubengewinde bilden; und
Umspritzen des ersten Gewindeabschnitts und dadurch Ausgestalten einer Mutter (12), die die Schraube umfasst.

## Revendications

1. Dispositif pour distribuer un matériau, comprenant un ensemble piston (10), l'ensemble piston ayant une vis comprenant une première partie filetée (111) et une seconde partie filetée (112), les première et seconde parties filetées diffèrent en forme et, en combinaison, forment un filetage de vis continu, l'ensemble piston comprenant en outre un écrou (12) qui vient en prise avec le filetage de vis par un filetage d'écrou, et dans lequel le filetage d'écrou correspond en forme à une forme négative de la première partie filetée, dans lequel la différence en forme est basée sur une structure de retenue dans la première partie filetée qui est absente dans la seconde partie filetée, et dans lequel la structure de retenue est fournie par un filetage conique formant au moins une partie de la première partie filetée.

2. Dispositif selon la revendication 1, dans lequel la structure de retenue est une bande qui fait saillie dans la rainure de filetage de la première partie filetée.

3. Dispositif selon l'une quelconque des revendications précédentes, dans lequel les première et seconde parties filetées sont basées sur le même pas et profil de filet, la seconde partie filetée étant formée par un filetage droit externe, et dans lequel le filetage de la première partie filetée et le filetage de la seconde partie filetée continuent l'un dans l'autre.

4. Dispositif selon l'une quelconque des revendications précédentes, dans lequel la vis a un joint adjacent à la première partie filetée et une poignée adjacente à la seconde partie filetée, dans lequel chacun du joint et de la poignée est plus grand en diamètre externe que le diamètre externe du filetage de vis.

5. Dispositif selon l'une quelconque des revendications précédentes, dans lequel chacun de la vis et de l'écrou est formé de manière monolithique.

6. Dispositif selon l'une quelconque des revendications précédentes, dans lequel la vis et l'écrou sont moulés à partir d'un matériau plastique et dans lequel l'écrou forme un surmoulage sur la vis.

7. Dispositif selon la revendication 6, dans lequel l'écrou est réalisé à partir d'un matériau renforcé de fibre de verre ayant une teneur en fibre de verre plus élevée en poids que la vis, et dans lequel la vis a de préférence une teneur en fibre de verre en poids de zéro.

8. Dispositif selon l'une quelconque des revendications précédentes, comprenant en outre un récipient ayant une extrémité avant avec une ouverture de distribution et une extrémité arrière opposée avec une ouverture arrière, et dans lequel l'ensemble piston étant fixé à l'écrou dans le récipient.

9. Dispositif selon la revendication 8, dans lequel l'écrou a un filetage externe venant en prise avec un filetage interne du récipient, dans lequel le récipient forme une chambre interne s'étendant entre l'ouverture de distribution et l'ouverture arrière, la chambre interne ayant une première partie de chambre et une seconde partie de chambre avec la seconde partie de chambre formant le filetage de récipient interne, dans lequel une marche est formée entre les première et seconde parties de chambre.

10. Procédé d'assemblage d'un dispositif de distribution de matériau, comprenant les étapes consistant à :
fournir le dispositif selon l'une quelconque des revendications précédentes ;
faire tourner l'ensemble piston (10) et/ou le récipient (20) l'un par rapport à l'autre et faire en sorte de ce fait que l'écrou (12) et le récipient (20) se vissent l'un dans l'autre ;
lorsque l'écrou et le récipient ne peuvent plus tourner lorsque l'écrou et la marche sont en contact l'un avec l'autre, tourner davantage l'ensemble piston et/ou le récipient l'un par rapport à l'autre et faire en sorte de ce fait que la vis et l'écrou se vissent l'un par rapport à l'autre.

11. Procédé de fabrication d'un ensemble piston selon l'une quelconque des revendications précédentes, comprenant les étapes consistant à :
mouler une vis comprenant une première partie filetée (111) et une seconde partie filetée (112), les première et seconde parties filetées diffèrent en forme mais forment en combinaison un filetage de vis continu ; et
surmouler la première partie filetée et former de ce fait un écrou (12) entourant la vis.
